# EUROPÄISCHE PATENTSCHRIFT

(11) **EP 3 284 505 B1**
(45) Veröffentlichungstag und Bekanntmachung des Hinweises auf die Patenterteilung: **27.03.2019**
(21) Anmeldenummer: 16184672.0
(22) Anmeldetag: 18.08.2016
(51) Int. Cl.: A61M 37/00, A61Q 1/14, A61K 8/365, A61K 8/41, A61K 8/02, A61K 8/19

(54) **VERFAHREN ZUM ENTFERNEN VON TÄTOWIERUNGEN UND VERWENDUNG EINES MICRONEEDLE-PATCHES IN EINEM VERFAHREN ZUM ENTFERNEN VON TÄTOWIERUNGEN**
METHOD OF REMOVING TATTOOS AND USE OF A MICRO NEEDLE PATCHES IN A METHOD FOR REMOVING TATTOOS
PROCEDE DESTINE A ENLEVER LES TATOUAGES ET UTILISATION D'UN PATCH A MICRO-AIGUILLE DANS UN PROCEDE DESTINE A ELIMINER DES TATOUAGES

(43) Veröffentlichungstag der Anmeldung: 21.02.2018
(73) Patentinhaber: nolax AG, 6203 Sempach Station (CH)
(72) Erfinder: Engel, Robertino, 5000 Aarau (CH); Lang, Christoph, 6207 Nottwil (CH)
(74) Vertreter: Hepp Wenger Ryffel AG

(56) Entgegenhaltungen:
- WO-A1-2010/051551
- US-A1- 2008 221 548
- US-A1- 2013 309 186
- US-A1- 2014 094 837

## Beschreibung

Die Erfindung betrifft ein Verfahren zum Entfernen von Tätowierungen und die Verwendung eines Microneedle-Patches in einem Verfahren zum Entfernen von Tätowierungen gemäss dem Oberbegriff der unabhängigen Ansprüche.

Tätowierungen werden derzeit aufgrund der meist zufrieden stellenden Ergebnisse mit Lasermethode entfernt. Dabei werden die in der Haut eingeschlossenen Farbpigmente durch die Laserbestrahlung so stark erhitzt, dass sie zerbersten. Laserbehandlungen können durch den Energieeintrag zu Schädigungen der obersten und umliegenden Hautschichten führen. Die verbleibenden Spaltprodukte der Farbpartikel werden dann vom Lymphsystem abtransportiert. Die Laserbestrahlung ist nachteilig, da sie teuer und aufwendig ist. Bei zu langer Exposition kann es jedoch auch zu Verbrennung der Haut kommen. Zudem sind viele Sitzungen notwendig, um eine Tätowierung zu entfernen. Des Weiteren muss für jede Farbe eine andere Wellenlänge des Lasers verwendet werden. Die Spaltprodukte sind gesundheitlich bedenklich, da sie durchaus kanzerogen sein können. Des Weiteren lagern sich die Spaltprodukte der Laserbehandlung oftmals in den Lymphknoten ab und verbleiben im Körper.

Als Alternative zur Laserentfernung von Tätowierungen existieren Verfahren mit flüssigen Entfernern. Hierbei handelt es sich um chemische Lösungen, meist Säuren, beispielsweise Milchsäure, welche auf oder in die Haut eingebracht werden. Diese Substanzen verursachen in der Haut eine Entzündung mit einer Wundbildung. Über diese Wunde scheidet der Körper ein Wundsekret mitsamt den in der Haut eingelagerten Pigmenten der Tätowierung aus. Diese chemischen Verfahren sind aggressiv für die behandelten Hautstellen und haben das Risiko, von Infektionen und weiterer Wundheilungsstörungen sowie Narbenbildung bei unsachgemässer Versorgung der Wunde.

Chemische Entferner werden beispielsweise mittels herkömmlichen Tätowiermaschinen in die Haut eingebracht oder als Creme auf die Haut aufgebracht. Das Einbringen mit einer Tätowiermaschine ist teuer, aufwendig und schädigt die Haut. Das Aufbringen als Creme führt meistens nicht zum erwünschten Erfolg und schädigt auch andere, nicht tätowierte Hautschichten aufgrund der oberflächlichen Anwendung. Die Epidermis wird dabei zerstört bzw. entfernt.

Im Stand der Technik ist das Einbringen von chemischen Entfernern in die Haut mittels Microneedle-Arrays beschrieben. WO 2010/051551 A1 offenbart das Auftragen von Microneedle-Arrays auf die Haut eines Patienten für therapeutische und präventive Behandlungen. Hierbei kann eine Tätowierung durch einen in die Haut eingebrachten Farbstoff überlagert oder durch ein eingebrachtes Bleichmittel aufgelöst werden.

WO 2015/027328 A1 beschreibt das Einbringen einer Zusammensetzung umfassend Bisphosphonate in einen tätowierten Hautbereich, welche ein Verblasen der Tätowierung verursacht. Die Zusammensetzung kann mittels Microneedle-basierten Injektions- und Infusionssystemen in intradermale Bereiche eingebracht werden.

US 2013/309186 A1, US 2008/221548 A1 und US 2014/094837 A1 beschreiben ebenfalls Verfahren zur Entfernung von Tätowierungen.

Nachteilig an den Verfahren im Stand der Technik ist, dass die Farbpigmente, deren Spaltprodukte und die chemischen Entferner in der Haut, bzw. im Körper, verbleiben und unter Umständen schädlich sind.

Es ist daher die Aufgabe der Erfindung, die Nachteile des Stands der Technik zu Überwinden. Insbesondere ist es eine Aufgabe der Erfindung ein Verfahren bereitzustellen, mit welchem auf einfache und kosteneffiziente Art und Weise Tätowierungen entfernt werden, sich die Farbpigmente und/oder eingebrachte chemische Entferner nicht im Körper ablagern und die Haut, bzw. der Körper, nicht zusätzlich geschädigt wird. Dies wird durch die Merkmale der unabhängigen Ansprüche erreicht.

Die Erfindung betrifft ein Verfahren zum Entfernen von Tätowierungen umfassend die Schritte Positionieren eines Microneedle-Patches auf einem tätowierten Bereich der Haut, wobei die Microneedles in die tätowierte Hautschicht eindringen, und Einbringen, insbesondere mittels manuellem Druck, einer insbesondere wässrigen Lösung zum Lösen der Farbpigmente durch die Microneedles des Patches in die Haut. Des Weiteren wirkt die eingebrachte Lösung für eine Dauer im Bereich von 15 bis 45 min, bevorzugt von 20 bis 40 min, besonders bevorzugt 25 bis 35 min ein. Die Farbpigmente werden durch die Microneedles des Patches mittels Unterdruck entfernt. Auf diese Weise verbleibt die eingebrachte Lösung nur zeitweise in der Haut. Da die eingebrachte Lösung und die Farbpigmente der Tätowierung entfernt werden, lagern sie sich nicht in der Haut bzw. im Körper ab. Eine Schädigung der Haut bzw. des Körpers durch verbleibende Substanzen wird minimiert oder ganz verhindert.

Das erfindungsgemässe Verfahren kann von Personal ohne spezielle medizinische Expertise ausgeführt werden. Eine derartige medizinische Expertise benötigt es nicht. Somit kann das Verfahren beispielsweise in Tätowierstudios oder Kosmetikstudios angeboten und durchgeführt werden.

Unter Lösen der Farbpigmente wird hier und im Folgenden verstanden, dass die Farbpigmente aus der Zellmatrix gelöst werden.

Die Microneedles sind hohl ausgebildet, um Lösungen einzubringen und zu entfernen. Dabei ist der Patch bevorzugt flexibel, d.h. nicht steif ausgebildet, und ist so an die Haut anpassbar.

Der Begriff "Patch" wird synonym für Array, Anordnung oder Pflaster verwendet. Der Patch kann selbstklebend oder nichtselbstklebend ausgestaltet sein.

Die Lösung zum Lösen der Farbpigmente kann einen pH von < 7, bevorzugt im Bereich von 0.5 bis 4, besonders bevorzugt im Bereich von 1 bis 3.5, aufweisen und vorzugsweise Milchsäure umfassen. Alternativ kann die Lösung einen pH von > 7, bevorzugt im Bereich von 10 bis 14, besonders bevorzugt im Bereich von 11 bis 13.5, aufweisen und vorzugsweise eine Base umfassend ausgewählt aus der Gruppe Calciumhydroxid, Natriumhydroxid, Kaliumhydroxid, Bariumhydroxid, Amine, bspw. Ehylendiamin oder Butylamine. Alternativ kann die Lösung einen pH im Bereich von 6.0 bis 8.0, bevorzugt im Bereich von 6.5 bis 7.5, und ein Enzym aufweisen. Derart werden die Farbpigmente der Tätowierung besonders gut aus der Zellmatrix der Haut gelöst. Bei der Verwendung eines Enzyms in der Lösung können auch pH Werte ausserhalb des Bereichs 6.0 bis 8.0 verwendet werden, sofern das Enzym in dem gewählten pH Wert funktional ist.

Sofern die Lösung Milchsäure umfasst, kann die Milchsäure in einer Konzentration im Bereich von 10 bis 40 Vol.-%, vorliegen. Der pH-Wert der Lösung liegt derart im sauren Bereich und kann insbesondere im Bereich von 0.5 bis 4, bevorzugt von 1 bis 3.5, liegen. Derart werden die Farbpigmente der Tätowierung besonders gut aus der Zellmatrix der Haut gelöst.

Eine bevorzugte Lösung umfasst 40 Vol.-% Milchsäure und weist einen pH-Wert von 1 bis 1.5 auf. Eine weitere bevorzugte Lösung umfasst 20 Vol.-% Milchsäure und weist einen pH-Wert von 2.5 bis 3 auf. Derartige Lösungen sind beispielsweise von Skinial® erhältlich.

Sofern die Lösung eine Base umfasst, liegt der pH-Wert der Lösung im alkalischen Bereich und kann bevorzugt im Bereich von 10 bis 14, bevorzugt 11 bis 13.5, liegen. Besonders bevorzugt ist der pH Wert 12.5. Bevorzugte Lösungen sind alkalisch, bevorzugt mit einem pH-Wert von 12.5. Derartige Lösungen sind beispielsweise unter dem Handelsnamen Rejuvi Tattoo Remover erhältlich. Derart werden die Farbpigmente der Tätowierung besonders gut aus der Zellmatrix der Haut gelöst.

Bevorzugt weist die Lösung falls aufweisend ein Enzym einen pH Wert im Bereich von 6.0 bis 8.0, bevorzugt im Bereich von 6.5 bis 7.5 auf.

Die Microneedles können eine Länge im Bereich von 200 bis 2000 µm, bevorzugt 300 bis 1700 µm, besonders bevorzugt 400 bis 1600 µm, ganz besonders bevorzugt 500 bis 1500 µm, aufweisen. Auf diese Weise reichen die Microneedles bei verwendungsgemässer Anwendung in die Hautschicht, in welcher sich die Farbpigmente der Tätowierung befinden. Derart wird die Lösung zum Lösen der Farbpigmente durch die Microneedles besonders zielgerichtet in tätowierte Hautbereiche eingebracht und nach dem Einwirken effizient entfernt.

Einzelne Microneedles des Patches können unterschiedliche Längen aufweisen. Derart können mit einem Microneedle-Patch Lösungen zum Entfernen der Farbpigmente in unterschiedliche Hautschichten eingebracht und entfernt werden. Dies erhöht die Effizienz der Entfernung einer Tätowierung, weil in unterschiedliche Hautschichten gleichzeitig appliziert und entfernt werden kann.

Die Microneedles weisen bevorzugt als Herstellungsmaterial Metall, Glass, Silizium oder Kunststoff auf.

Zum Entfernen der Farbpigmente wird ein Unterdruck an die Microneedles angelegt. Dieser Unterdruck ist bevorzugt im Bereich von 200 bis 500 mmHg.

Der Unterdruck wird bevorzugt für eine Dauer von 1 bis 10 Stunden, bevorzugt 2 bis 8 Stunden, besonders bevorzugt 3 bis 6 Stunden, angelegt und entsprechend lange extrahiert. Derart werden die Farbpartikel der Tätowierung vollständig aus dem tätowierten Hautbereich entfernt.

Ein weiterer Aspekt der Erfindung betrifft die Verwendung eines Microneedle-Patches in einem Verfahren zum Entfernen von Tätowierungen wie vorgängig erläutert.

Bevorzugt weist das Microneedle-Patch eine Grösse von 9 cm², 25 cm² oder 100 cm² auf. Das Microneedle-Patch kann rechteckig bspw. 3 cm x 3 cm, 5 cm x 5 cm oder 10 cm x 10 cm, ausgestaltet sein. Ebenso möglich sind runde Ausgestaltungen. Die Microneedle-Patches weisen bevorzugt neun Microneedles in einem Bereich von 4 mm².

Ein Kit kann ein oder mehrere Microneedle-Patches und eine oder mehrere Lösungen zum Lösen von Farbpigmenten eines tätowierten Bereichs aufweisen. Derart kann der Anwender je nach gewünschter Anwendung eine Lösung zum Lösen von Farbpigmenten auswählen. Optional kann das kann das

Kit Anwendungshinweise, insbesondere zur Ausführung eines Verfahrens wie vorgängig erläutert, aufweisen. Alternativ kann das Kit mehrere Microneedle-Patches aufweisen, welche unterschiedliche Lösungen zum Lösen von Farbpigmenten eines tätowierten Bereichs enthalten. Derart kann der Anwender je nach gewünschter Anwendung ein Microneedle-Patches enthaltend eine Lösung zum Lösen von Farbpigmenten auswählen.

### AUSFÜHRUNGSBEIPIELE

Das erfindungsgemässe Verfahren wurde an einem Stück Schweinehaut getestet. Die Haut wurde am toten Schwein exzidiert. Die Schweinehaut wurde zunächst bereichsweise tätowiert. 24 Stunden nach Beendigung des Tätowierens wurde ein Microneedle-Patch auf der tätowierten Schweinehaut positioniert, wobei die Microneedles in die tätowierte Hautschicht eindrangen. Das Microneedle-Patch weist pro 4 mm² neun Microneedles auf. Das Microneedle-Patch enthält in einem Reservoir eine Lösung enthaltend 20 Vol.-% Milchsäure mit einem pH-Wert von 2.5 bis 3 zum Lösen der Farbpigmente. Das Reservoir steht in Fluidverbindung mit den Microneedles. Mittels manuellem Druck wurde die Milchsäure-Lösung durch die Microneedles des Patches in den tätowierten Bereich der Haut eingebracht. Die Lösung wirkte für 30 min ein. Zum Entfernen der Farbpigmente wurde eine Pumpe über einer Verbindung, hier einem Schlauch, mit dem Microneedle-Patch verbunden und ein Unterdruck von 350 mmHg angelegt. Die Farbpigmente, welche durch das Einwirken der Milchsäure gelöst wurden, wurden mittels des Unterdrucks extrahiert. Die Extraktion erfolgte für 5 Stunden.

Nachfolgend wird die Erfindung anhand von Figuren näher beschrieben.

Es zeigen:
- Figur 1:: Ein Flussdiagramm des erfindungsgemässen Verfahrens;
- Figur 2:: Schnittskizze eines Microneedle-Patches für die Entfernung von Tätowierungen.

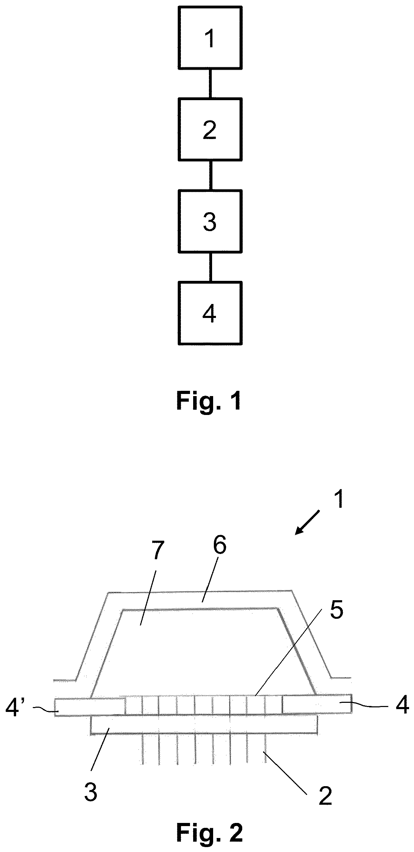

Figur 1 zeigt die Verfahrensschritte des erfindungsgemässen Verfahrens zum Entfernen von Tätowierungen. In Schritt 1 wird ein Microneedle-Patches auf einem tätowierten Bereich der Haut positioniert, wobei die Microneedles in die tätowierte Hautschicht eindringen. Dabei durchdringen die Microneedles Hautschichten, welche sich oberhalb einer tätowierten Hautschicht befinden, und bilden Kanäle in die tätowierte Hautschicht. Unter der tätowierten Hautschicht wird die Hautschicht eines tätowierten Bereichs einer Haut verstanden, in welchem sich die Farbpigmente der Tätowierung befinden.

In Schritt 2 wird eine Lösung zum Lösen der Farbpigmente durch die Microneedles des Patches in die Haut eingebracht. Dies erfolgt bevorzugt mittels manuellem Druck.

Die eingebrachte Lösung wirkt für eine Dauer von 30 min ein (Schritt 3). Derart werden die Farbpigmente aus der Zellmatrix gelöst und können anschliessend entfernt werden.

In Schritt 4 werden die, insbesondere gelösten, Farbpigmente mittels Unterdruck durch die Microneedles des Patches extrahiert und so entfernt. Hierzu ist das Microneedle-Patch mit einer Vorrichtung zum Erzeugen eines Unterdrucks verbunden. Dazu wird die Vorrichtung zum Erzeugen eines Unterdrucks über einen Schlauch direkt mit dem Microneedle-Patch verbunden. Alternativ kann das Microneedle-Patch entfernt und ein zweites Microneedle-Patch für die Extraktion auf die Haut aufgebracht werden. An das Microneedle-Patch wird ein Unterdruck von 350 mmHG angelegt und für eine Dauer von 5 h extrahiert. Alternativ kann ein Pflaster auf das Microneedle-Patch und umliegende Hautbereich dichtend aufgebracht werden. Das Pflaster ist hierbei über einen Schlauch mit der Vorrichtung zum Erzeugen eines Unterdrucks verbunden. Wie vorgängig erläuterte kann ein Unterdruck angelegt und derart die Farbpigmente extrahiert werden.

Figur 2 zeigt schematisch ein Microneedle-Patch 1 für die Verwendung im erfindungsgemässen Verfahren. Das Microneedle-Patch 1 weist Microneedles 2 auf, welche durch ein Substrat 3 hindurchragen. Die Microneedles 2 und das Substrat 3 bilden einen sogenannten Microneedle-Chip. Das Substrat 3 ist bereichsweise mit einer Hotmelt-Schicht 4 verklebt. Die Microneedles 2 stehen in Kontakt mit einer Membran 5. Die Membran 5 steht in Kontakt mit der Hotmelt-Schicht 4. Die Hotmelt-Schicht 4 ist an einem backing layer 6 angeordnet. Die Membran 5 und der backing layer 6 bilden ein Reservoir 7 für die Aufnahme der Lösung zum Entfernen einer Tätowierung. Das Microneedle-Patch 1 kann entsprechend im Reservoir 7 die Lösung zum Entfernen einer Tätowierung aufweisen. Die Membran 5 hält die Lösung im Reservoir zurück. Die Membran 5 ist permeable für die Lösung, sobald Druck auf das Reservoir 7 ausgeübt wird. Derart kann die Lösung durch manuellen Druck in die Haut eingebacht werden.

Das Microneedle-Patch 1 wird auf einen tätowierten Bereich der Haut positioniert, beispielsweise mittels der Hotmelt-Schicht 4 aufgeklebt, so dass die Microneedles 2 die oberen Hautschichten durchstechen und in Hautschichten hineinragen, in welcher sich Farbpigmente der Tätowierung befinden. Durch die hohle Ausgestaltung der Microneedles 2 bilden diese Kanäle zwischen dem Reservoir 7 und der tätowierten Hautschicht. Die Lösung zum Entfernen kann durch die Microneedles 2 in den tätowierten Bereich eingebracht werden. Nach einer Einwirkzeit im Bereich von 30 min werden die, insbesondere gelösten, Farbpigmente über die Microneedles 2 mittels Unterdruck extrahiert.

## Patentansprüche

1. Verfahren zum Entfernen von Tätowierungen umfassend die Schritte
- Positionieren eines Microneedle-Patches auf einem tätowierten Bereichs der Haut, wobei die Microneedles in die tätowierte Hautschicht eindringen;
- Einbringen, insbesondere mittels manuellem Druck, einer insbesondere wässrigen Lösung zum Lösen der Farbpigmente durch die Microneedles des Patches in die Haut;
- Einwirken der eingebrachten Lösung für eine Dauer im Bereich von 15 bis 45 min, bevorzugt von 20-40 min, besonders bevorzugt 25 bis 35 min;
**dadurch gekennzeichnet, dass** das Entfernen der Farbpigmente durch Extrahieren mittels Unterdruck durch die Microneedles des Patches erfolgt.

2. Verfahren nach Anspruch 1, wobei die Lösung zum Lösen der Farbpigmente
- einen pH von < 7, bevorzugt im Bereich von 0.5 bis 4, besonders bevorzugt im Bereich von 1 bis 3.5, aufweist und vorzugsweise Milchsäure umfasst; oder
- einen pH von > 7, bevorzugt im Bereich von 10 bis 14, besonders bevorzugt im Bereich von 11 bis 13.5, aufweist und vorzugsweise umfassend eine Base ausgewählt aus der Gruppe Calciumhydroxid, Natriumhydroxid, Kaliumhydroxid, Bariumhydroxid, Amine, bspw. Ehylendiamin oder Butylamine;
- oder einen pH im Bereich von 6.0 bis 8.0, bevorzugt im Bereich von 6.5 bis 7.5, und ein Enzym aufweist.

3. Verfahren nach Anspruch 2, wobei die Milchsäure in einer Konzentration im Bereich von 5 bis 45 Vol.-%, bevorzugt 10 bis 40 Vol.-%, vorliegt.

4. Verfahren nach einem der Ansprüche 1 bis 3, wobei die Microneedles eine Länge im Bereich von 200 bis 2000 µm, bevorzugt 300 bis 1700 µm, besonders bevorzugt 400 bis 1600 µm, ganz besonders bevorzugt 500 bis 1500 µm, aufweisen.

5. Verfahren nach einem der Ansprüche 1 bis 4, wobei einzelne Microneedles des Patches unterschiedliche Längen aufweisen.

6. Verwendung eines Microneedle-Patches in einem Verfahren zum Entfernen von Tätowierungen nach einem der Ansprüche 1 bis 5.

## Claims

1. Method for removing tattoos, comprising the steps of
- positioning a microneedle patch on a tattooed area of the skin, wherein the microneedles penetrate into the tattooed skin layer;
- introducing, especially by means of manual pressure, into the skin through the microneedles of the patch an especially aqueous solution for dissolving the color pigments;
- allowing action of the introduced solution for a period within the range from 15 to 45 min, preferably from 20-40 min, particularly preferably from 25 to 35 min;
**characterized in that** the color pigments are removed by extraction by means of negative pressure through the microneedles of the patch.

2. Method according to Claim 1, wherein the solution for dissolving the color pigments
- has a pH of < 7, preferably within the range from 0.5 to 4, particularly preferably within the range from 1 to 3.5, and preferably comprises lactic acid; or
- has a pH of > 7, preferably within the range from 10 to 14, particularly preferably within the range from 11 to 13.5, and preferably comprising a base selected from the group consisting of calcium hydroxide, sodium hydroxide, potassium hydroxide, barium hydroxide, amines, for example ethylenediamine or butylamines;
- or has a pH within the range from 6.0 to 8.0, preferably within the range from 6.5 to 7.5, and an enzyme.

3. Method according to Claim 2, wherein the lactic acid is present in a concentration within the range from 5 to 45% by volume, preferably from 10 to 40% by volume.

4. Method according to any of Claims 1 to 3, wherein the microneedles have a length within the range from 200 to 2000 µm, preferably from 300 to 1700 µm, particularly preferably from 400 to 1600 µm, very particularly preferably from 500 to 1500 µm.

5. Method according to any of Claims 1 to 4, wherein individual microneedles of the patch have differing lengths.

6. Use of a microneedle patch in a method for removing tattoos according to any of Claims 1 to 5.

## Revendications

1. Procédé d'élimination de tatouages, comprenant les étapes suivantes :
- le positionnement d'un patch à microaiguilles sur une zone tatouée de la peau, les microaiguilles pénétrant dans la couche de peau tatouée ;
- l'introduction, notamment au moyen d'une pression manuelle, d'une solution notamment aqueuse pour la dissolution des pigments colorés au travers des microaiguilles du patch dans la peau ;
- l'action de la solution introduite pendant une durée dans la plage allant de 15 à 45 minutes, de préférence de 20 à 40 minutes, de manière particulièrement préférée de 25 à 35 minutes ;
**caractérisé en ce que** l'élimination des pigments colorés a lieu par extraction au travers des microaiguilles du patch au moyen d'une sous-pression.

2. Procédé selon la revendication 1, dans lequel la solution pour la dissolution des pigments colorés
- présente un pH < 7, de préférence dans la plage allant de 0,5 à 4, de manière particulièrement préférée dans la plage allant de 1 à 3,5, et comprend de préférence de l'acide lactique ; ou
- présente un pH > 7, de préférence dans la plage allant de 10 à 14, de manière particulièrement préférée dans la plage allant de 11 à 13,5, et comprend de préférence une base choisie dans le groupe constitué par l'hydroxyde de calcium, l'hydroxyde de sodium, l'hydroxyde de potassium, l'hydroxyde de baryum, les amines, par exemple l'éthylène-diamine ou les butylamines ;
- ou présente un pH dans la plage allant de 6,0 à 8,0, de préférence dans la plage allant de 6,5 à 7,5, et comprend une enzyme.

3. Procédé selon la revendication 2, dans lequel l'acide lactique est présent en une concentration dans la plage allant de 5 à 45 % en volume, de préférence de 10 à 40 % en volume.

4. Procédé selon l'une quelconque des revendications 1 à 3, dans lequel les microaiguilles présentent une longueur dans la plage allant de 200 à 2 000 µm, de préférence de 300 à 1 700 µm, de manière particulièrement préférée de 400 à 1 600 µm, de manière tout particulièrement préférée de 500 à 1 500 µm.

5. Procédé selon l'une quelconque des revendications 1 à 4, dans lequel des microaiguilles individuelles du patch présentent des longueurs différentes.

6. Utilisation d'un patch à microaiguilles dans un procédé d'élimination de tatouages selon l'une quelconque des revendications 1 à 5.
